# EUROPEAN PATENT SPECIFICATION

(11) **EP 3 603 595 B1**
(45) Date of publication and mention of the grant of the patent: **11.08.2021**
(21) Application number: 18770445.7
(22) Date of filing: 06.03.2018
(51) Int. Cl.: A61F 13/534, A61F 13/532

(54) **ABSORBENT PAD**
SAUGFÄHIGE UNTERLAGE
TAMPON ABSORBANT

(30) Priority: 21.03.2017 JP 2017055039
(43) Date of publication of application: 05.02.2020
(73) Proprietor: Daio Paper Corporation, Ehime 799-0492 (JP)
(72) Inventor: SUYAMA, Junnosuke, Sakura-shi Tochigi 329-1411 (JP)
(74) Representative: Bandpay & Greuter
(86) International application number: PCT/JP2018/008474
(87) International publication number: WO 2018/173737

(56) References cited:
- EP-A1- 0 481 322
- WO-A1-2013/094726
- WO-A1-2013/094726
- GB-A- 2 135 893
- JP-A- H0 549 658
- JP-A- H0 549 658
- JP-A- 2006 297 073
- JP-A- 2016 526 983
- US-A- 3 968 798
- US-A- 4 029 101

## Description

### TECHNICAL FIELD

The present invention relates to absorbent pads used for absorbent articles such as disposable diapers and sanitary napkins and pads.

### BACKGROUND ART

Traditionally, an absorbent pad is known which is formed by folding inward both ends in the width direction of an absorbent pad sheet comprising a liquid-permeable front sheet, a liquid-permeable rear sheet and absorbent polymers in between the front sheet and the liquid-permeable rear sheet (Patent Document 1).

There is also an absorbent pad which is formed by folding inward both ends in the width direction of an absorbent pad sheet comprising a liquid-permeable front sheet, a liquid-permeable rear sheet, and absorbent polymers and pulp fibers in between the front sheet and the liquid-permeable rear sheet, and further, by folding outward the inner parts of the folded both ends (Patent Document 2). JPH05-49658 also discloses such an absorbent pad configured as a disposable diaper.

### CITATION LIST

### Patent Document

[Patent Document 1] Japanese Unexamined Patent Publication No. 2004-500165
[Patent Document 2] Japanese Unexamined Patent Publication No. 2013-132460

### SUMMARY OF INVENTION

### Technical Problem

However, the absorbent pad according to Patent Document 1 carries a risk of leaking excess liquid excretions to outside which have not been absorbed into the absorbent pad since a slit-like spacing is formed in between the both ends folded inward.

Further, the absorbent pad according to Patent Document 2 gets thicker by being folded, that may give discomforts to a wearer.

In view of the foregoing, the objective of the invention is to provide an absorbent pad that prevents the leakage of the excess liquid excretions not being absorbed by the absorbent pad to outside.

### Solution to Problem

The above objective is achieved by the invention disclosed as follows:
A first aspect of the present invention is directed to an absorbent pad formed by folding inward both ends in a width direction of an absorbent pad sheet. The absorbent pad sheet is formed with a liquid-permeable front sheet, a liquid-permeable rear sheet and absorbent polymer particles absorbing liquid excretions that are provided in between the front sheet and the rear sheet. A width in the width direction between the both ends folded inward is formed 10 ∼ 50 % with respect to a width in the width direction of the absorbent pad, and a part of the absorbent polymer particles in sections folded inward is escaped down to inside of the front sheet in the sections folded inward. A base weight of the front sheet is greater than a base weight of the rear sheet.

According to a second aspect of the present invention, as in the embodiment of the first aspect, when viewed in plan, the front sheet and the rear sheet are joined by first junctions extending in a longitudinal direction at predetermined intervals in the width direction and second junctions extending in the width direction at predetermined intervals in the longitudinal direction. and the absorbent polymer particles are packed within a plurality of cells defined by the first junctions and the second junctions.

According to a third aspect of the present invention, as in the embodiment of the first or second aspect, a thickness of the front sheet is formed thicker than a thickness of the rear sheet.

According to a fourth aspect of the present invention, as in the embodiment of any one of the first, second, and third aspects, a base weight of the absorbent polymer particles in the sections folded inward is arranged greater than a base weight of the absorbent polymer particles in sections other than the sections folded inward of the absorbent pad sheet.

### ADVANTAGES OF THE INVENTION

According to the first aspect, the absorbent pad is formed with a liquid-permeable front sheet, a liquid-permeable rear sheet and absorbent polymer particles absorbing the liquid excretions that are provided in between the front sheet and the rear sheet, the width in the width direction between the both ends folded inward is formed 10 ∼ 50 % with respect to the width in the width direction of the absorbent pad, and a part of the absorbent polymer particles in the sections folded inward is escaped down to the inside of the front sheet in the sections folded inward. Therefore, it is possible to prevent the leakage of the liquid excretions to the outside by absorbing plenty of the liquid excretions diffusing toward the width direction at the both ends of the absorbent pad, and further, to prevent a gel blocking phenomenon by distributing the absorbent polymer particles also within the front sheet. In addition, since pulp fibers are not used, the absorbent pad may be formed thin and it is possible to reduce the discomforts of the wearer. As the base weight of the front sheet is greater than the base weight of the rear sheet, it is possible to adjust the amount of the absorbent polymer particles escaping down into the front sheet in the sections folded inward to a predetermined amount.

According to the second aspect, when viewed in plan, the front sheet and the rear sheet are joined by first junctions extending in the longitudinal direction at predetermined intervals in the width direction and second junctions extending in the width direction at predetermined intervals in the longitudinal direction, and the absorbent polymer particles are packed within a plurality of cells defined by the first junctions and the second junctions. Therefore, in addition to the effect of the first aspect, the liquid excretions transferred to the center in the width direction of the absorbent pad can be quickly diffused to the both ends of the absorbent pad. In addition, it is possible to further prevent a gel blocking phenomenon of the absorbent polymer particles by suppressing uneven distribution of the absorbent polymer particles.

According to the third aspect, the thickness of the front sheet is thicker than the thickness of the rear sheet. Therefore, in addition to the effect of the first or second aspect, it is possible to adjust the amount of the absorbent polymer particles escaping down into the front sheet in the sections folded inward to a predetermined amount.

According to the fourth aspect, the base weight of the absorbent polymer particles in the sections folded inward is greater than the base weight of the absorbent polymer particles in the sections other than the sections folded inward of the absorbent pad sheet. Therefore, in addition to the effects of any one of the first, second, and third aspects, it is possible to prevent the leakage of the liquid excretions to the outside by absorbing even more of the liquid excretions diffusing toward the width direction at the both ends of the absorbent pad.

### BRIEF DESCRIPTION OF THE DRAWINGS

FIG. 1 is an expansion diagram showing the inner surface plan view of the disposable diapers.
FIG. 2 is an expansion diagram showing the outer surface plan view of the disposable diapers.
FIG. 3 is a cross sectional diagram showing A-A in FIG. 1.
FIG. 4 is a cross sectional diagram showing B-B in FIG. 1.
FIG. 5 is expansion diagrams showing the absorbent pad of the first embodiment and (a) shows the inner surface plan view and (b) shows a cross sectional diagram of A-A.
FIG. 6 is expansion diagrams showing the absorbent pad of the first embodiment right before folding and (a) shows the inner surface plan view and (b) shows a cross sectional diagram of A-A.
FIG. 7 is diagrams showing the absorbent pad of the first embodiment and (a) shows the inner surface plan view and (b) shows a cross sectional diagram of A-A.
FIG. 8 is an explanatory diagram showing the folded left side of the absorbent pad of the first embodiment.
FIG. 9 is expansion diagrams showing the absorbent pad of the second embodiment right before folding and (a) shows the inner surface plan view and (b) shows a cross sectional diagram of A-A.
FIG. 10 is an explanatory diagram showing the folded left side of the absorbent pad of the second embodiment.
FIG. 11 is a diagram showing other folding embodiment.

### DESCRIPTION OF EMBODIMENTS

### < Disposable diapers >

The invention disclosing an absorbent pad with the excellent fluids diffusion and the excellent liquid permeability is described while referring to the figures. As used herein, the term "the longitudinal direction" refers to the direction connecting the stomach side and the back side, the term "the width direction" refers to the direction perpendicular to the longitudinal direction, the term "vertical direction" refers to the direction perpendicular to the waistline direction of disposable diapers in a worn state, the term "inner surface" refers to the body side surface of each parts, and the term "outer surface" refers to the non-body side surface of each parts.

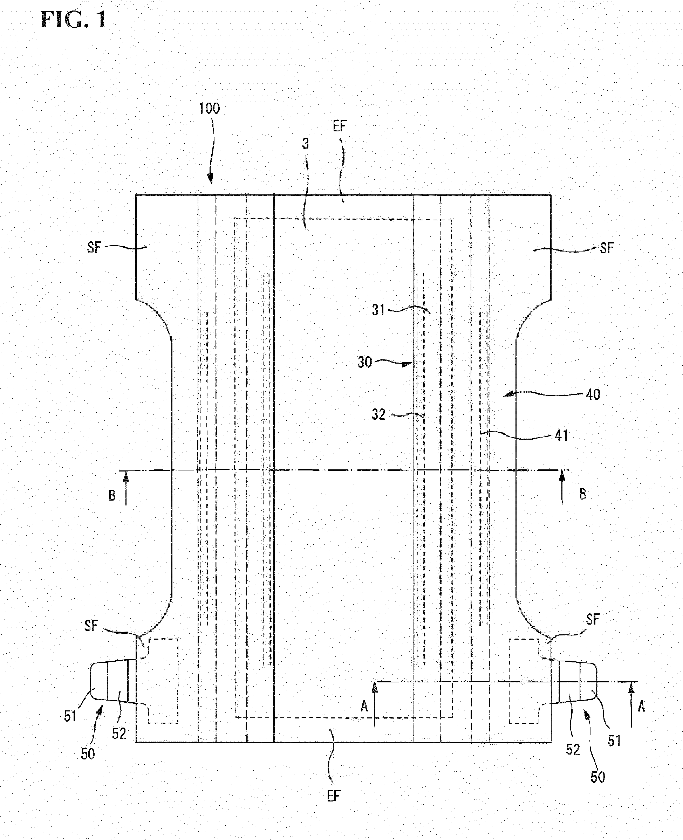

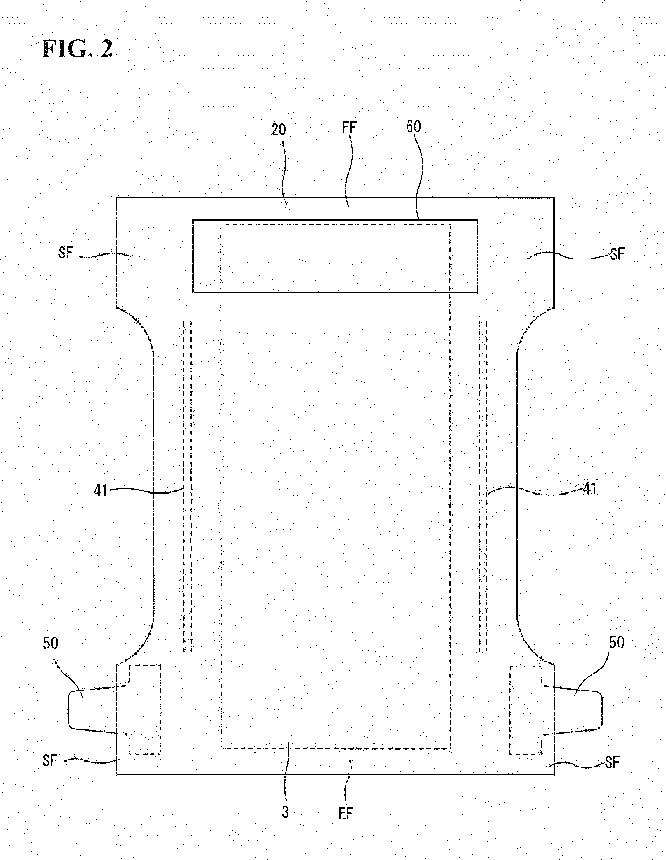

As shown in FIGS. 1 and 2, a disposable diaper 100 comprises a liquid-permeable top sheet 1 on the body side, a liquid-impermeable back sheet 2 on the non-body side and an absorbent pad 3 positioned in between the top sheet 1 and the back sheet 2. Further, the exterior sheet 20 is provided on the outer surface of the back sheet 2.

Three-dimensional gathers 30 for the leg circumference are respectively provided on outer portion of the absorbent pad 3 in the width direction thereof to prevent the leakage of the liquid excretions to outside, and flat gathers 40 for the leg circumference preventing the leakage of the liquid excretions to outside are respectively provided outside of the three-dimensional gathers 30.

End flaps EF, where the absorbent pad 3 does not extend, are respectively provided at portions outside the absorbent pad 3 in the longitudinal direction thereof, and side flaps SF, where the absorbent pad 3 does not extend, are respectively provided at portions outside the absorbent pad 3 in the width direction thereof.

Fastening tapes 50 extending outward in the width direction are respectively provided at the back side of the side flaps SF, and a target sheet 60 for locking the fastening tapes 50 when wearing the disposable diaper 100 is provided at the stomach side of the outer surface of the exterior sheet 20.

### (Top sheet)

The top sheet 1 extends outside the outer peripheral line of the absorbent pad 3, and the outer surface of the extending part is fixed to the inner surface of the back sheet 2 through an adhesive agent such as a hot melt.

Porous or nonporous non-woven fabric or perforated plastic sheet may be used for the top sheet 1. For the material fibers constituting the non-woven fabric, synthetic fibers, for example olefins such as polyethylene or polypropylene, polyesters, and amide-based, regenerated fibers such as rayon or cupro, as well as natural fibers such as cotton may be used. Further, as for processing methods of non-woven fabric, the known methods such as a spun lace method, a spun bond method, a SMS method, a thermal bond method, a melt-blown method, a needle punching method, an air-through method, and a point bond method may be used. A fiber base weight of the non-woven fabric used for the top sheet 1 is preferably 15 ∼ 30g/m² and the thickness is preferably 0.05 ∼ 1 mm.

### (Back sheet)

The back sheet 2 extends outside the outer peripheral line of the absorbent pad 3 and blocks the movement of the liquid excretions absorbed in the absorbent pad 3 to outside.

As the back sheet 2, in addition to plastic films such as polyethylene films, moisture permeable sheets while keeping the water impermeable property from the viewpoint of the stuffiness prevention may be used. Microporous sheets obtained by forming the sheets by melting and kneading inorganic fillers in olefin-based resins such as polyethylene or polypropylene, followed by stretching them toward the uniaxial or biaxial direction may be used for such sheets with the water barrier property and the moisture permeability, for example. A base weight per unit area of the back sheet 2 is preferably 13 ∼ 40g/m² and the thickness is preferably 0.01 ∼ 0.1 mm.

### (Absorbent pad)

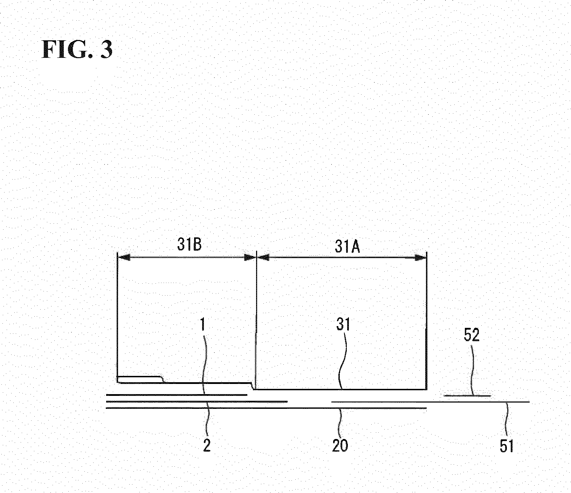

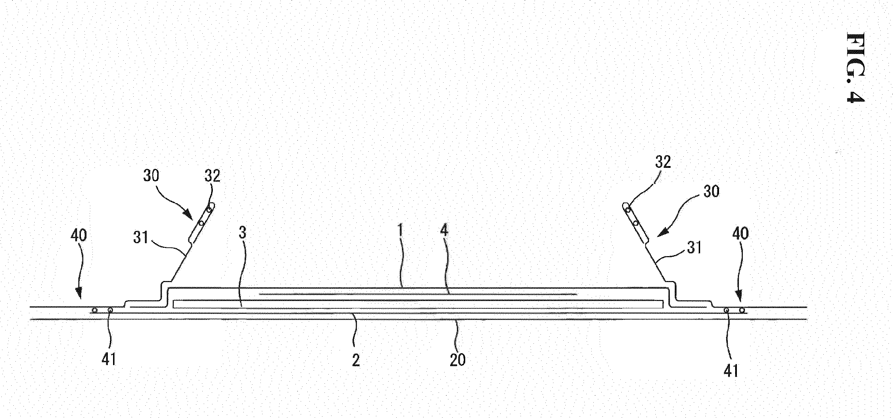

As shown in FIGS. 3, 4 and 7, the absorbent pad 3 is formed by folding a front sheet 71 of a left folded section A1 of the absorbent pad sheet 70 toward the inside in the width direction so as to face a front sheet 71 of a left section A2, and by folding a front sheet 71 of a right folded section A5 of the absorbent pad sheet 70 toward the inside in the width direction so as to face a front sheet 71 of the right section A2. The absorbent pad sheet 70 will be described later.

### (Exterior sheet)

The exterior sheet 20 is a sheet that covers the outer surface of the back sheet 2 and provides the outer surface of the disposable diaper 100 with a fabric-like appearance and texture. The exterior sheet 2 0 is preferably formed with non-woven fabric. As for the material fibers, synthetic fibers, for example olefins such as polyethylene or polypropylene, polyesters, and amide-based, regenerated fibers such as rayon or cupro, as well as natural fibers such as cotton may be used. As for the processing methods, for example a spun lace method, a spun bond method, a thermal bond method, an air-through method and a needle punching method may be used for the production. However, in the viewpoint of establishing compatibility between texture and strength, long fiber non-woven fabric such as spun bond non-woven fabric, SMS non-woven fabric or SMMS non-woven fabric is preferred.

In addition to the use of non-woven fabric in one piece, it is possible to use overlapping multiple sheets. Preferably the non-woven fabric is fixed with each other by applying an adhesive agent such as a hot melt when using overlapping multiple sheets. Further, the fiber base weight is preferably 10 ∼ 50 g/m², more preferably 15 ∼ 30 g/m² when using a non-woven fabric.

### (Three-dimensional gather for the leg circumference)

The outer surface of a base 31A of the gather sheet 31 forming the three-dimensional gather 30 for the leg circumference is fixed to the outer part of the inner surface of the back sheet 2 in the width direction and the outer part of the inner surface of the exterior sheet 20 in the width direction respectively, throughout the longitudinal direction. Further, the both ends of raised portions 31B of the gather sheet 31 in the longitudinal direction are fixed to the outer part of the inner surface of the top sheet 1 in the width direction, and the middle part of the raised portions 31B of the gather sheet 31 in the longitudinal direction is not fixed to the inner surface of the top sheet 1 but separated.

A plurality of elongated elastic stretchable strips 32 extending in the longitudinal direction at the predetermined intervals in the width direction are provided in a predetermined extended state at the raised portions 31B of the gather sheet 31. Accordingly, when wearing the disposable diaper 100, the raised portions 31B is raised toward the crotch part of the wearer by the shrinkage force of the elastic stretchable strips 32, and the leakage of the liquid excretions to outside can be prevented by pressing and contacting the tip of the raised portions 31 against the crotch part of the wearer.

As for the gather sheet 31, in addition to the non-woven fabric such as spun bond non-woven fabric, plastic films similar to the ones used for the back sheet 2 or laminated sheets thereof may be used. However, in the viewpoint of feeling to the skin, water repellent non-woven fabric is preferred.

For the elastic stretchable strips 32, materials commonly used such as thread-like, string-like or belt-like natural rubbers or synthetic rubbers, specifically styrene-based rubbers, olefin rubbers, urethane rubbers, ester-based rubbers, polyurethanes, polyethylenes, polystyrenes, styrene- butadienes, silicones and polyesters may be used. Further, the thickness of the elastic stretchable strips 32 is approximately 500 ∼ 1500 dtex, especially around 800 ∼ 1300 dtex (in case of natural rubbers, approximately 0.1 ∼ 3 mm, especially around 0.5 ∼ 3 mm) is preferred, and the elongation percentage at the time of installation is approximately 150 ∼ 250 %, especially around 160 ∼ 200 % is preferred.

### (Flat gather for the leg circumference)

The flat gather 40 for the leg circumference is provided at the base 31A of the gather sheet 31 forming the three-dimensional gather 30. Elongated elastic stretchable strips 41 extending in the longitudinal direction at the predetermined intervals in the width direction are provided in a predetermined extended state at the base 31A of the gather sheet 31 and the outer part of the back sheet 2 in the width direction which form the side flaps SF. Accordingly, when wearing the disposable diaper 100, the flat gather 40 is pressed and contacted to the leg part of the wearer by the shrinkage force of the elastic stretchable strips 41, and the leakage of the liquid excretions to outside can be prevented.

For the elastic stretchable strips 41, materials commonly used such as thread-like, string-like or belt-like natural rubbers or synthetic rubbers, specifically styrene-based rubbers, olefin rubbers, urethane rubbers, ester-based rubbers, polyurethanes, polyethylenes, polystyrenes, styrene-butadienes, silicones and polyesters may be used. In addition, the spacing between the elastic stretchable strips 41 is approximately 2 ∼ 15 mm, especially around 3 ∼ 7 mm is preferred. Further, the thickness of the elastic stretchable strips 41 is approximately 500 ∼ 1500 dtex, especially around 800 ∼ 1300 dtex (in case of natural rubbers, approximately 0.1 ∼ 3 mm, especially around 0.5 ∼ 3 mm) is preferred, and the elongation percentage at the time of installation is approximately 150 ∼ 250 %, especially around 160 ∼ 200 % is preferred.

### (Fastening tape)

As shown in FIGS. 1 ∼ 3, the fastening tapes 50 extending toward outside are provided at the back side of the side flaps SF respectively. The fastening tape 50 comprises a base sheet 51 and a locking section 52 provided at the outer part of the inner surface of the base sheet 51. Further, the inner part of the base sheet 51 is fixed to the outer part of the gather sheet 31 and the exterior sheet 20 in the width direction.

The non-woven fabric is preferred for the material of the base sheet 51 and any known non-woven fabric may be used without a particular limitation. For the material fibers constituting the non-woven fabric, synthetic fibers, for example olefins such as polyethylene or polypropylene, polyesters, and amide-based, regenerated fibers such as rayon or cupro as well as natural fibers such as cotton may be used. Further, for the processing methods of non-woven fabric, the known methods such as a spun lace method, a spun bond method, a SMS method, a thermal bond method, a melt-blown method, a needle punching method, an air-through method, and a point bond method may be used. Especially, the spun bond non-woven fabric and SMS non-woven fabric using olefin fibers are preferred. Although the base weight of the non-woven fabric used may be appropriately determined, the total base weight of the non-woven fabric for the main part 5b is 20∼ 75 g/m², preferably 26 ∼ 46 g/m², and the total base weight of the non-woven fabric for the fixed part 5f and the tip part 5p is respectively 35 ∼ 130 g/m², preferably 46 ∼ 116 g/m². Within this range, it is possible to ensure the strength and the rigidity of the base section fixed in between the exterior sheet 20 and the gather sheet 31, and further secure the flexibility and the elasticity of the main unit 74.

For the locking section 52, hook materials of a mechanical fastener are preferred. The hook materials may have multiple engaging protrusions. The configuration of the engaging protrusions may be in shapes such as (A) a mirror-inverted J shape, (B) J-shape, (C) mushroom-shape, (D) T-shape and (E) a dual J shape (a combination of two J shapes joined together back to back like a fishhook), but may be in any shape. Alternatively, it is possible to use pressure-sensitive adhesive layers instead of the hook materials.

### (Target sheet)

For the target sheet 60, plastic films with a plurality of loop yarn on the surface side or non-woven fabrics may be preferably used. Thus, when a wearer wears the disposable diaper 100, it is possible to efficiently lock the locking section 52 of the fastening tapes 50 to the target sheet 60.

### (Interlayer sheet)

In the first embodiment, an interlayer sheet 4 is provided in between the top sheet 1 and the absorbent pad 3. Thus, the liquid excretions passing through the top sheet 1 can move quickly to the absorbent pad 3 and it is possible to prevent the liquid excretions from returning to the top sheet 1. It is noted that the interlayer sheet 4 is fixed to the outer surface of the top sheet 1 by a hot melt adhesive agent, heat embossing or ultrasonic wave welding.

For the interlayer sheet 4, in addition to the non-woven fabrics, resin films with numerous permeation holes may be used. For the non-woven fabrics, materials similar to the top sheet 1 may be used. However, materials having higher hydrophilicity and higher fiber density than the top sheet 1 are preferred in order to have excellent movement properties of the liquid excretions from the top sheet 1 to the interlayer sheet 4.

### < Absorbent pad sheet >

### (First embodiment)

The absorbent pad sheet 70 in the first embodiment forming the absorbent pad 3 is described herein.

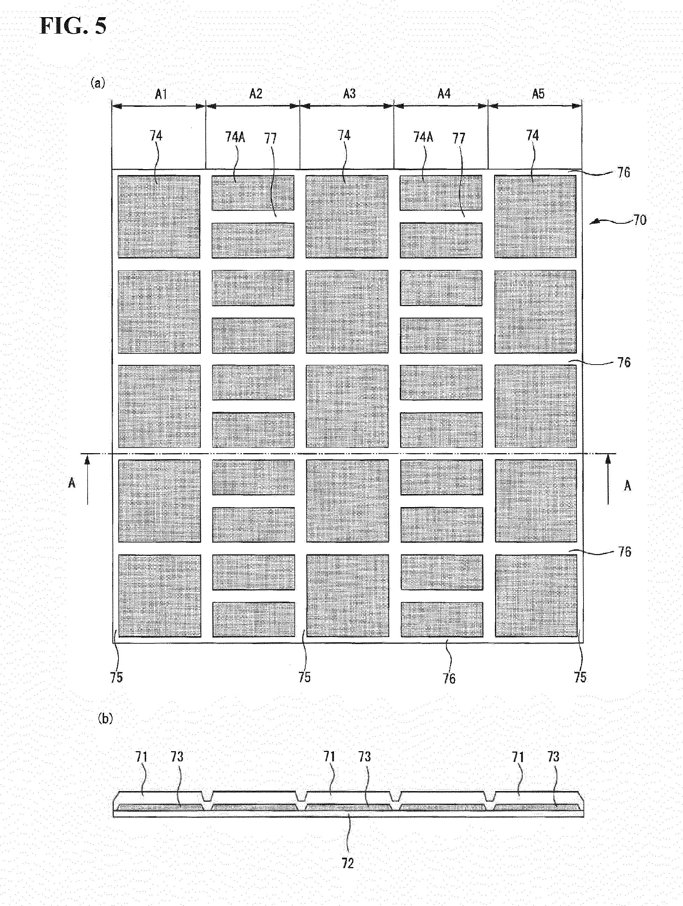

As shown in FIG. 5, the absorbent pad sheet 70 is formed with a liquid-permeable front sheet 71 on the top sheet 1 side, a liquid-permeable rear sheet 72 on the back sheet 2 side and absorbent polymer particles 73 provided in between the front sheet 71 and the rear sheet 72.

The front sheet 71 and the rear sheet 72 are joined by junctions and the absorbent polymer particles 73 are provided respectively in a plurality of cells 74 defined by the junctions. It is noted that the front sheet 71 and the rear sheet 72 positioned at the cells 74 are not joined but separated to form predetermined spaces. Thus, it is possible to prevent the absorbent polymer particles 73 from being unevenly distributed in one part of the absorbent pad sheet 70 and to suppress an occurrence of a gel blocking phenomenon. Further, the absorbent polymer particles 73 may not be bonded to the front sheet 71 or the rear sheet 72, or may be bonded to the front sheet 71 or the rear sheet 72 by using for example a hot melt adhesive agent.

The junctions are formed with 6 of the junctions 75 ("first junctions" recited in claims) extending toward the longitudinal direction at the predetermined intervals in the width direction of the absorbent pad sheet 70 and 6 of the junctions 75 ("second junctions" recited in claims) extending toward the width direction at the predetermined intervals in the longitudinal direction of the absorbent pad sheet 70. As used herein, counting from left to right, the term "left folded section A1" refers to a section defined by the first junction 75 and the second junction 75, the term "left section A2" refers to a section defined by the second junction 75 and the third junction 75, the term "center section A3" refers to a section defined by the third junction 75 and the fourth junction 75, the term "right section A4" refers to a section defined by the fourth junction 75 and the fifth junction 75 and the term "right folded section A5" refers to a section defined by the fifth junction 75 and the sixth junction 75.

When viewed in plan, the cells 74 formed in the left folded section A1 are configured in approximately squares, the cells 74 formed in the left section A2 are separated in the longitudinal direction by the junctions 77 extending in the width direction, and such separated small cells 74A are configured in approximately rectangles having the long side in the width direction. The cells 74 formed in the center section A3 are configured in approximately squares, the cells 74 formed in the right section A4 are separated in the longitudinal direction by the junctions 77 extending in the width direction, and such separated small cells 74A are configured in approximately rectangles having the long side in the width direction. The cells 74 formed in the right folded section A5 are configured in approximately squares.

Porous or nonporous non-woven fabric may be used for the front sheet 71. When using a non-woven fabric, a non-woven fabric processed by an air-through method, a melt-blown method or a needle punching method is preferred. Further, it is preferred to make the fiber diameter of the non-woven fabric used for the front sheet 71 thicker than the fiber diameter used for the rear sheet 72, to make the fiber base weight of the non-woven fabric of the front sheet 71 greater than the fiber base weight of the non-woven fabric used for the rear sheet 72, and to make the thickness of the non-woven fabric of the front sheet 71 thicker than the thickness of the non-woven fabric used for the rear sheet 72. Therefore, it is possible to efficiently absorb the liquid excretions diffusing toward the width direction since a predetermined amount of the absorbent polymer particles 73 provided in the cells 74 is transferred to the inside of the left folded section A1 folded inward of the left section A2 of the absorbent pad sheet 70. Similarly, it is possible to efficiently absorb the liquid excretions diffusing toward the width direction since a predetermined amount of the absorbent polymer particles 73 provided in the cells 74 is transferred to the inside of the right folded section A5 folded inward of the right section A4 of the absorbent pad sheet 70. The fiber diameter of the non-woven fabric used for the front sheet 71 is preferably 3.0 ∼ 30 dtex, the fiber base weight is preferably 20 ∼ 100 g/m² and the thickness is preferably 2 ∼ 5 mm.

Porous or nonporous non-woven fabric may be used for the rear sheet 72. When using a non-woven fabric, a high fiber density non-woven fabric processed by a spun bond method, a melt-blown method or a needle punching method is preferred. Therefore, it is possible to minimize the escape of the absorbent polymer particles 73 from the cells 74. The fiber diameter of the non-woven fabric used for the rear sheet 72 is preferably 1.0 ∼ 3.0 dtex, the fiber base weight is preferably 10 ∼ 20 g/m² and the thickness is preferably 0.2 ∼ 2 mm.

High absorbent polymer particles used for absorption items such as disposable diapers and sanitary napkins may be used for the absorbent polymer particles 73. The high absorbent polymer particles may be, for example, starch, cellulose and synthetic polymers, and starch-acrylic acid (salt) graft copolymer, saponified product of starch - acrylonitrile copolymer, crosslinked products of sodium carboxymethylcellulose or acrylic acid (salt) polymer.

The water absorption of the absorbent polymer particles 73 is preferably more than or equal to 40 g/g and the water absorption rate is preferably less than or equal to 70 seconds, more preferably less than or equal to 40 seconds. Accordingly, it is possible to efficiently absorb the liquid excretions passing through the Top Sheet 1 with the absorbent pad sheet 70 and to prevent the liquid excretions from returning to the Top Sheet 1.

The gel strength of the absorbent polymer particles 73 is preferably more than or equal to 1,000 Pa. Accordingly, it is possible to reduce the stickiness of the absorbent pad sheet 70 which absorbed the liquid excretions.

The particle diameter of the absorbent polymer particles 73 is preferably, when sifting (shaking for 5 minutes) by using a 500 µm standard sieve (JISZ8801-1:2006) and sifting (shaking for 5 minutes) the particles falling under said sieve with said sifting by using a 180 µm standard sieve (JISZ8801-1:2006), such that the ratio of the particles remaining on the 500 µm standard sieve is less than or equal to 30 wt. % and the ratio of the particles remaining on the 180 µm standard sieve is more than or equal to 60 wt. %.

The fiber base weight of the absorbent polymer particles 73 of the cells 74 may b e determined appropriately depending on the absorption amount required, but preferably 50 ∼ 350 g/m². It is noted that the fiber base weight less than 50 g/m² makes it difficult to ensure the absorption amount, and the fiber base weight more than 350 g/m² results in excessive absorption amount. The configuration of the cells 74 may be formed in squares in the plan view, however, it may be formed in shapes of rectangles, rhombus, hexagon, circle, and ellipse as well. Furthermore, when the cells 74 are formed in hexagonal shapes, the junctions 75 are formed extending in the longitudinal direction while following in a staggered manner in the width direction along the shapes of hexagons in the width direction, and the junctions 76 are formed extending in the width direction while following in a staggered manner in the longitudinal direction along the shapes of hexagons in the longitudinal direction.

The width in the width direction of the junctions 75 is formed in 5 ∼ 10 mm, the width in the longitudinal direction of the junctions 76 is formed in 5 ∼ 10 mm and the width in the longitudinal direction of the junctions 77 is formed in 5 ∼ 10 mm. Therefore, the liquid excretions passing through the top sheet 1 can be spread throughout the entire absorbent pad sheet 70 and the liquid excretions can be absorbed efficiently with the plurality of cells 74.

The junctions 75, the junctions 76 and the junctions 77 are preferably bonded by welding the front sheet 71 and the rear sheet 72, for example by ultrasonic wave welding or heat sealing. However, a hot melt adhesive agent may also be used for bonding.

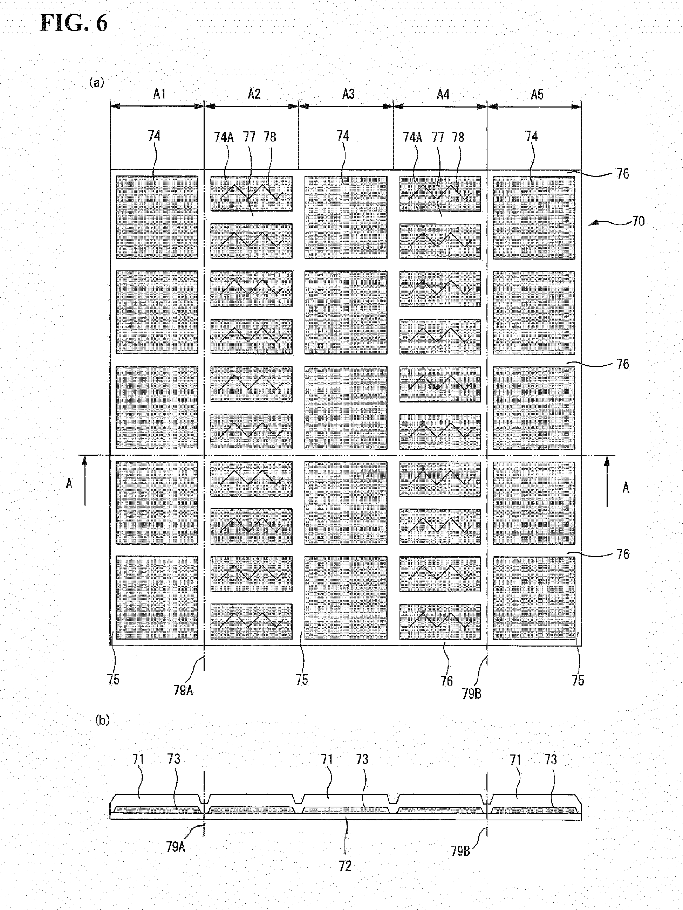

Next, a technique forming the absorbent pad 3 by folding the absorbent pad sheet 70 is described. As shown in FIG. 6, first, the hot melt adhesive agent 78 is applied to the front sheet 71 positioned in the left section A2 and the right section A4 of the absorbent pad sheet 70. It is noted that the hot melt adhesive agent 78 is preferably applied in a spiral shape or a striped shape in order to maintain a high liquid permeability.

Next, having a center on a folding line 79A extending toward the longitudinal direction at approximately the center in the width direction of the junction 75 in between the left folded section A1 and the left section A2, the left folded section A1 is folded inward, and the front sheet 71 positioned in the left folded section A1 is fixed to the front sheet 71 positioned in the left section A2 by applying the hot melt adhesive agent 78. Similarly, having a center on a folding line 79B extending toward the longitudinal direction at approximately the center in the width direction of t h e junction 75 in between the right folded section A5 and the right section A4, the right folded section A5 is folded inward, and the front sheet 71 positioned in the right folded section A5 is fixed to the front sheet 71 positioned in the right section A4 by applying the hot melt adhesive agent 78. It is noted that, instead of the hot melt adhesive agent 78, a thermocompression bonding or an ultrasonic wave welding may be used to fix the left folded section A1 with the left section A2 and the right folded section A5 with the right section A4.

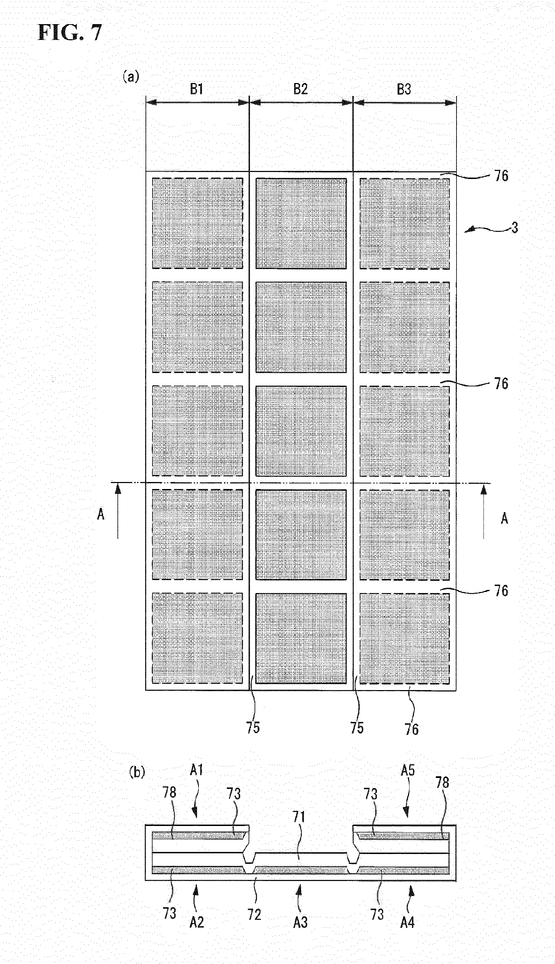

Next, the absorbent pad 3 formed by folding the absorbent pad sheet 70 is described. As shown in FIG. 7, the left section B1 and the right section B3 of the absorbent pad 3 in the width direction are formed in a convex configuration toward inside (the body side) with respect to the center section B2 of the absorbent pad 3 in the width direction. Thus, it is possible to reduce the discomforts of the wearer by preventing the liquid excretions not absorbed in the center section B2 of the absorbent pad 3 (hereinafter referred to the excess liquid excretions) from contacting the wearer's buttock, for example.

The width of the center section B2 of the absorbent pad 3 in the width direction is preferably 10 ∼ 40% with respect to the width of the absorbent pad 3 in the width direction. There is a risk that the liquid excretions passing through the top sheet 1 may be transferred to the left section B1 or the right section B3 of the absorbent pad 3 if the width of the center section B2 is less than 10%. Further, there is a risk of not being able to absorb all the excess liquid excretions diffused in the width direction if the width of the center section B2 is more than 40%.

The junctions 76 extending toward the width direction are formed in the left section B1 and the right section B3 of the absorbent pad 3. Therefore, it is possible to quickly diffuse the excess liquid excretions to the left section Bland the right section B3 of the absorbent pad 3 and to suppress an occurrence of a gel blocking phenomenon. In addition, the junctions 77 extending toward the width direction are further formed in the left section A2 forming the outside (non-body side) of the left section B1 and the right section A4 forming the outside of the right section B3 of the absorbent pad 3. Thus, it is possible to more quickly diffuse plenty of the excess liquid excretions to the left section B1 and the right section B3 of the absorbent pad 3 by the junctions 76 and the junctions 77, making the absorption rate faster, and to prevent the leakage to the outside. Furthermore, the excess liquid excretions diffused to the left section B1 of the absorbent pad 3 are absorbed in the left folded section A1 and the left section A2 forming the left section B1, and the excess liquid excretions diffused to the right section B3 of the absorbent pad 3 are absorbed in the right folded section A5 and the right section A4 forming the right section B3, thus enabling rewetting phenomenon to be reduced and discomforts for the wearer to be reduced.

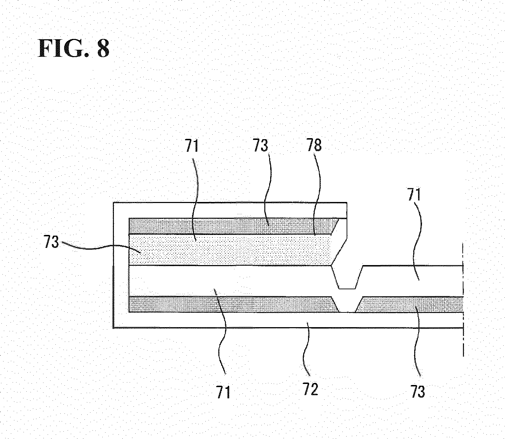

As shown in FIG. 8, the absorbent polymer particles 73 escaped down from the cells 74 are distributed in the left folded section A1 of the front sheet 71 forming the left section B1 and the right folded section A5 of the front sheet 71 forming the right section B3 of the absorbent pad 3. Thus, it is possible to prevent the leakage to the outside by absorbing the excess liquid excretions diffused to the left section Bland the right section B3 of the absorbent pad 3.

### (Second embodiment)

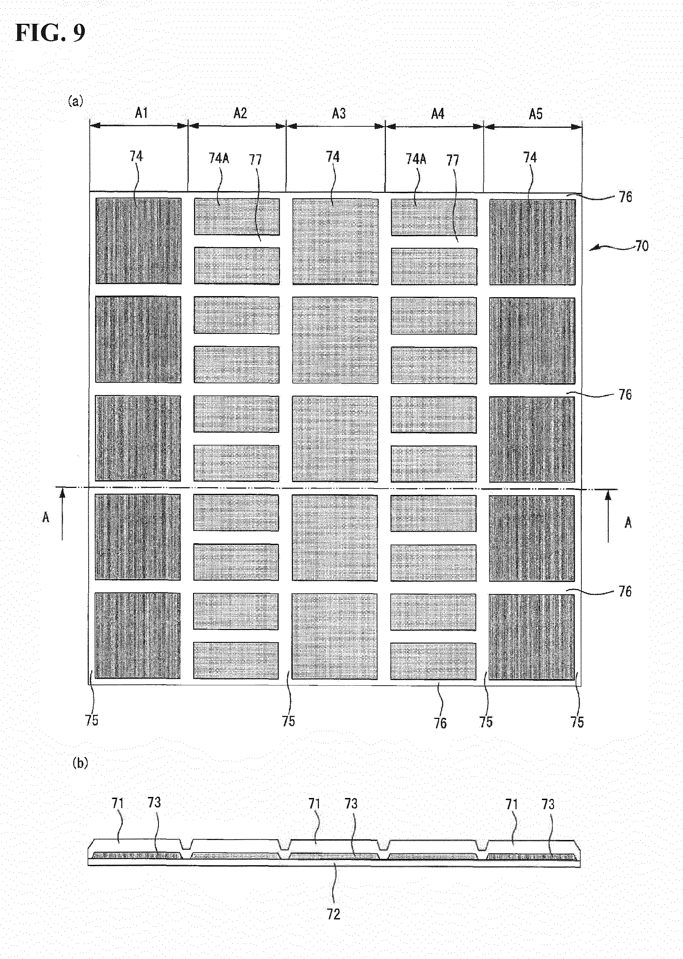

The absorbent pad sheet 70 of the second embodiment forming the absorbent pad 3 is described. It is noted that the same members as the absorbent pad sheet 70 of the first embodiment are described with the same reference numerals and the explanations are not repeated. As shown in FIG. 9, in the absorbent pad sheet 70 of the second embodiment, the base weight of the absorbent polymer particles 73 provided in the cells 74 formed in the left folded section A1 and the right folded section A5 of the absorbent pad sheet 70 is set to 150% with respect to the base weight of the absorbent polymer particles 73 provided in the cells 74 formed in the left section A2, the center section A3 and the right section A4 of the absorbent pad sheet 70. It is noted that the base weight of the absorbent polymer particles 73 is differentiated in the absorbent pad sheet 70 of the second embodiment, however, a material, amount of the liquid absorption, a gel strength or a particle size of the absorbent polymer particles 73 may be differentiated as well.

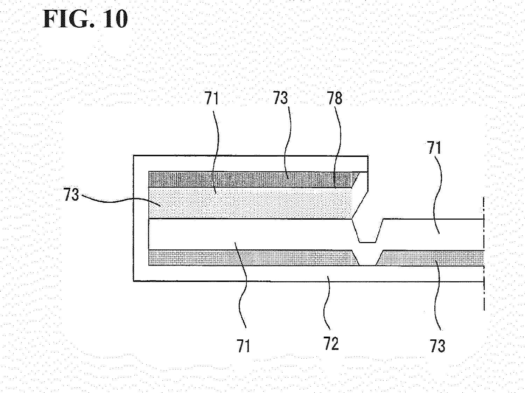

As shown in FIG. 10, plenty of absorbent polymer particles 73 escaped down from the cells 74 are distributed in the left folded section A1 of the front sheet 71 forming the left section B1 and the right folded section A5 of the front sheet 71 forming the right section B3 of the absorbent pad 3 formed by folding the absorbent pad sheet 70 of the second embodiment. Thus, it is possible to prevent the leakage to the outside by absorbing more excess liquid excretions diffusing to the left section B1 and the right section B3 of the absorbent pad 3.

### (Other folding embodiment)

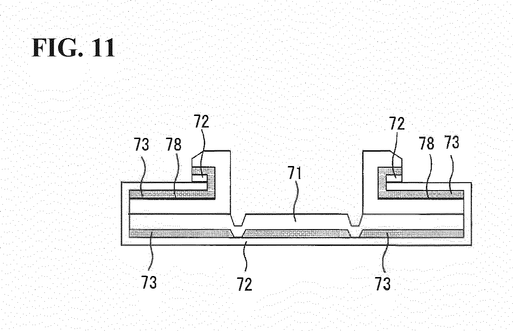

Next, the other folding embodiment is described. It is noted that the same components as the absorbent pad sheet 70 of the first embodiment are described with the same reference numerals and the explanations are not repeated. As shown in FIG. 11, the inside part in the width direction of the left folded section A1 forming the left section B1 of the absorbent pad 3 may be folded outward and the inside part in the width direction of the right folded section A5 forming the right section B3 of the absorbent pad 3 may be folded outward. Thus, it is possible to reduce even more discomforts of the wearer by preventing more liquid excretions from contacting the wearer's buttock, for example.

### INDUSTRIAL APPLICABILITY OF INVENTION

The present invention may be applied to absorbent articles such as tape type disposable diapers, pants type disposable diapers and sanitary napkins and pads.

### DESCRIPTION OF REFERENCE CHARACTERS

- 70: Absorbent pad sheet
- 71: Front sheet
- 72: Rear sheet
- 73: Absorbent polymer particles
- 74: Cells
- 75: Junctions (first junctions)
- 76: Junctions (second junctions)

## Claims

1. An absorbent pad (3) formed by folding inward both ends in a width direction of an absorbent pad sheet (70), wherein:
the absorbent pad sheet is formed with a liquid-permeable front sheet (71), a liquid-permeable rear sheet (72) and absorbent polymer particles (73) absorbing liquid excretions that are provided in between the front sheet and the rear sheet;
a width in the width direction between the both ends folded inward is formed 10 ~ 50 % with respect to a width in the width direction of the absorbent pad;
a part of the absorbent polymer particles in sections folded inward is escaped down to inside of the front sheet in the sections folded inward; and
a base weight of the front sheet is formed greater than a base weight of the rear sheet.

2. The absorbent pad of claim 1, wherein:
when viewed in plan, the front sheet and the rear sheet are joined by first junctions extending in a longitudinal direction at predetermined intervals in the width direction and second junctions extending in the width direction at predetermined intervals in the longitudinal direction; and
the absorbent polymer particles are packed within a plurality of cells defined by the first junctions and the second junctions.

3. The absorbent pad of claim 1 or 2, wherein a thickness of the front sheet is formed thicker than a thickness of the rear sheet.

4. The absorbent pad of any one of claims 1 to 3, wherein a base weight of the absorbent polymer particles in the sections folded inward is arranged greater than a base weight of the absorbent polymer particles in sections other than the sections folded inward of the absorbent pad sheet.

## Patentansprüche

1. Absorbierendes Pad (3), das durch Einwärtsfalten beider Enden einer Bahn (70) absorbierenden Pads in einer Breiterichtung gebildet ist, wobei:
die Bahn absorbierenden Pads mit einer flüssigkeitsdurchlässigen vorderen Bahn (71), einer flüssigkeitsdurchlässigen hinteren Bahn (72) und absorbierenden Polymerpartikeln (73) gebildet ist, welche Flüssigkeitsausscheidungen absorbieren und zwischen der vorderen Bahn und der hinteren Bahn bereitgestellt sind,
eine Breite in der Breiterichtung zwischen beiden einwärts gefalteten Enden 10 - 50 % in Bezug auf eine Breie in der Breiterichtung des absorbierenden Pads gebildet ist,
ein Teil der absorbierenden Polymerpartikel in einwärts gefalteten Abschnitten nach unten zur Innenseite der vorderen Bahn in den einwärts gefalteten Abschnitten entwichen ist und
ein Flächengewicht der vorderen Bahn größer als ein Flächengewicht der hinteren Bahn gebildet ist.

2. Absorbierendes Pad nach Anspruch 1, wobei:
die vordere Bahn und die hintere Bahn in der Draufsicht durch erste Verbindungsstellen verbunden sind, die sich in festgelegten Intervallen in der Breiterichtung in der Längsrichtung erstrecken, und zweite Verbindungsstellen, die sich in festgelegten Intervallen in der Längsrichtung in der Breiterichtung erstrecken, und
die absorbierenden Polymerpartikel in mehrere Zellen gepackt sind, die durch die ersten Verbindungsstellen und die zweiten Verbindungsstellen definiert sind.

3. Absorbierendes Pad nach Anspruch 1 oder 2, wobei eine Dicke der vorderen Bahn größer als eine Dicke der hinteren Bahn gebildet ist.

4. Absorbierendes Pad nach einem der Ansprüche 1 bis 3, wobei ein Flächengewicht der absorbierenden Polymerpartikel in den einwärts gefalteten Abschnitten größer angeordnet ist als ein Flächengewicht der absorbierenden Polymerpartikel in anderen Abschnitten als den einwärts gefalteten Abschnitten der Bahn absorbieren Pads.

## Revendications

1. Tampon absorbant (3) formé en repliant vers l'intérieur les deux extrémités, dans le sens de la largeur, d'une feuille de tampon absorbant (70), dans lequel :
la feuille de tampon absorbant est formée avec une couche avant perméable au liquide (71), une couche arrière perméable au liquide (72) et des particules de polymère absorbant (73) qui absorbent les excrétions liquides qui se trouvent entre la couche avant et la couche arrière ;
une largeur, dans le sens de la largeur entre les deux extrémités repliées vers l'intérieur, est formée comme étant égale à 10 à 50% par rapport à une largeur dans le sens de la largeur du tampon absorbant ;
une partie des particules de polymère absorbant dans les sections repliées vers l'intérieur s'échappe vers l'intérieur de la couche avant dans les sections repliées vers l'intérieur ; et
un poids de base de la couche avant est formé comme étant supérieur à un poids de base de la couche arrière.

2. Tampon absorbant selon la revendication 1, dans lequel :
sur une vue de dessus, la couche avant et la couche arrière sont jointes par des premières jonctions qui s'étendent dans une direction longitudinale à des intervalles prédéterminés dans le sens de la largeur et des secondes jonctions qui s'étendent dans le sens de la largeur à des intervalles prédéterminés dans la direction longitudinale ; et
les particules de polymère absorbant sont contenues dans une pluralité de cellules définies par les premières jonctions et les secondes jonctions.

3. Tampon absorbant selon la revendication 1 ou 2, dans lequel une épaisseur de la couche avant est supérieure à une épaisseur de la couche arrière.

4. Tampon absorbant selon l'une quelconque des revendications 1 à 3, dans lequel un poids de base des particules de polymère absorbant dans les sections repliées vers l'intérieur est supérieur à un poids de base des particules de polymère absorbant dans les sections autres que les sections repliées vers l'intérieur de la feuille de tampon absorbant.
